# EUROPEAN PATENT APPLICATION

(11) **EP 4 257 983 A1**
(43) Date of publication of application: **11.10.2023**
(21) Application number: 22912781.6
(22) Date of filing: 29.03.2022
(51) Int. Cl.: G01N 33/72

(54) **GLYCATED HEMOGLOBIN MEASUREMENT KIT**

(30) Priority: 21.02.2022 KR 20220022049
(71) Applicant: Leobio Co., Ltd., Suwon-si, Gyeonggi-do 16648 (KR)
(72) Inventor: PARK, Kyung Won, Seongnam-si Gyeonggi-do 13203 (KR); MOON, Chang Sang, Hwaseong-si Gyeonggi-do 18484 (KR); JEON, Bo Ram, Hwaseong-si Gyeonggi-do 18412 (KR); SEO, Hyunju, Suwon-si Gyeonggi-do 16402 (KR); IM, Hye Rin, Suwon-si, Gyeonggi-do 16660 (KR); HWANG, Soonhye, Yongin-si Gyeonggi-do 16816 (KR); KIM, Jeongsoo, Suwon-si, Gyeonggi-do 16351 (KR)
(74) Representative: HGF
(86) International application number: PCT/KR2022/004464
(87) International publication number: WO 2023/158013

(57) **Abstract**

Disclosed is a glycated hemoglobin measurement kit including a reagent container that has a cylindrical shape that is open at opposite sides and that includes a barrier membrane that blocks an opening at one side of the reagent container and forms a reagent storage part that accommodates a reagent for measurement of glycated hemoglobin in blood, a cleaning solution container that has a cylindrical shape that is open at opposite sides and that includes an auxiliary barrier membrane that blocks an opening at one side of the cleaning solution container and forms a cleaning solution storage part that accommodates a cleaning solution that cleans a reaction solution in which the blood and the reagent are mixed and react with each other, and a container holder including an attachment/detachment part from which a measurement strip that measures the glycated hemoglobin is detachable and a measurement hole through which one area of the measurement strip attached to the attachment/detachment part is exposed, the reagent container or the cleaning solution container being selectively attached to or detached from the container holder to selectively provide the reaction solution or the cleaning solution toward the measurement hole.

## Description

### [TECHNICAL FIELD]

Embodiments of the inventive concept described herein relate to a glycated hemoglobin measurement kit.

### [BACKGROUND ART]

Diabetes is a metabolic disease in which blood glucose is higher than a normal range for a long period of time. When blood glucose remains high, acute complications such as diabetic ketoacidosis and hyperglycemic hyperosmolar non-ketotic coma and long-term complications such as cardiovascular disease, stroke, chronic kidney disease, diabetic ulcer, and diabetic retinopathy may occur. Due to this, a diabetic patient requires continuous management to prevent various complications by regularly checking blood glucose and to maintain a blood glucose level at an appropriate level. Accordingly, blood glucose is an important indicator for diagnosis or management of diabetes.

Blood glucose refers to the concentration of glucose contained in blood, and a blood glucose level is changed every moment under the influence of factors, such as diet and a physical activity state, without being fixed. Therefore, it is important to recognize average blood glucose rather than instantaneous blood glucose. A method of measuring a glycated hemoglobin (HbA1c) level is most widely used to recognize long-term blood glucose.

Glycated hemoglobin (HbA1c) refers to a form in which glucose in a blood vessel, that is, blood glucose is combined with hemoglobin normally present in a red blood cell. When a blood glucose level is high, the amount of blood glucose combined with hemoglobin increases, and therefore glycated hemoglobin increases. Furthermore, because combined glucose is not used and remains combined with hemoglobin, glycated hemoglobin remains at an average level. Meanwhile, because the average lifespan of red blood cells is about three months, glycated hemoglobin reflects the average blood glucose level for about three months.

However, blood glucose measurement devices are widely distributed to individuals and allow self-measurement at home, whereas glycated hemoglobin measurement devices are not widely distributed so that people have the inconvenience of having to visit medical institutions to measure glycated hemoglobin.

### [DETAILED DESCRIPTION OF THE INVENTION]

### [TECHINICAL PROBLEM]

Embodiments of the inventive concept provide a glycated hemoglobin measurement kit that enables a user to measure glycated hemoglobin by mixing blood, a reagent, and a cleaning solution without a visit to a medical institution and that is easy to carry.

The problems to be solved by the inventive concept are not limited to the aforementioned problems, and any other problems not mentioned herein will be clearly understood from the following description by those skilled in the art to which the inventive concept pertains.

### [TECHNICAL SOLUTION]

According to an embodiment, a glycated hemoglobin measurement kit includes a reagent container that has a cylindrical shape that is open at opposite sides and that includes a barrier membrane that blocks an opening at one side of the reagent container and forms a reagent storage part that accommodates a reagent for measurement of glycated hemoglobin in blood, a cleaning solution container that has a cylindrical shape that is open at opposite sides and that includes an auxiliary barrier membrane that blocks an opening at one side of the cleaning solution container and forms a cleaning solution storage part that accommodates a cleaning solution that cleans a reaction solution in which the blood and the reagent are mixed and react with each other, and a container holder including an attachment/detachment part from which a measurement strip that measures the glycated hemoglobin is detachable and a measurement hole through which one area of the measurement strip attached to the attachment/detachment part is exposed, the reagent container or the cleaning solution container being selectively attached to or detached from the container holder to selectively provide the reaction solution or the cleaning solution toward the measurement hole.

The glycated hemoglobin measurement kit may further include a capillary having a needle that collects the blood or breaks the barrier membrane, the capillary being attached to or detached from the reagent container or selectively attached to or detached from the container holder.

The glycated hemoglobin measurement kit may further include a breaking member having an auxiliary needle that breaks the auxiliary barrier membrane, the breaking member being attached to or detached from the cleaning solution container.

The glycated hemoglobin measurement kit may further include a stopper provided on the capillary and supported on a periphery of an opening at an opposite side of the reagent container to limit a movement of the capillary.

The glycated hemoglobin measurement kit may further include a guide groove that is formed in the reagent container through cutting and that guides a movement of the stopper such that the capillary moves toward the barrier membrane and breaks the barrier membrane.

The glycated hemoglobin measurement kit may further include an auxiliary stopper provided on the breaking member and supported on a periphery of an opening at an opposite side of the cleaning solution container to limit a movement of the breaking member.

The glycated hemoglobin measurement kit may further include an auxiliary guide groove that is formed in the cleaning solution container through cutting and that guides a movement of the auxiliary stopper such that the breaking member moves toward the auxiliary barrier membrane and breaks the auxiliary barrier membrane.

The glycated hemoglobin measurement kit may further include a pair of housings in which the reagent container or the cleaning solution container is selectively accommodated so as to be detachable and a base that supports the pair of housings and the container holder.

The glycated hemoglobin measurement kit may further include first alignment parts formed to protrude from, or concavely formed on, inner circumferential surfaces of the housings and the container holder along central axis directions of the housings and the container holder and second alignment parts formed to protrude from, or concavely formed on, outer circumferential surfaces of the reagent container and the cleaning solution container, the second alignment parts being engaged with the first alignment parts.

Other specific details of the inventive concept are included in the detailed description and the drawings.

### [ADVANTAGEOUS EFFECTS OF THE INVENTION]

According to an embodiment of the inventive concept, the glycated hemoglobin measurement kit enables the user to measure the glycated hemoglobin by mixing the blood, the reagent, and the cleaning solution without a visit to a medical institution and is easy to carry.

Effects of the inventive concept are not limited to the aforementioned effects, and any other effects not mentioned herein will be clearly understood from the following description by those skilled in the art to which the inventive concept pertains.

### [DESCRIPTION OF THE DRAWINGS]

FIG. 1 is a perspective view illustrating a glycated hemoglobin measurement kit according to an embodiment of the inventive concept;
FIG. 2 is a sectional view illustrating the glycated hemoglobin measurement kit according to an embodiment of the inventive concept;
FIGS. 3 to 10 are perspective views illustrating the glycated hemoglobin measurement kit according to an embodiment of the inventive concept; and
FIGS. 11 and 12 are perspective views illustrating a glycated hemoglobin measurement device according to an embodiment of the inventive concept.

### [BEST MODE]

The above and other aspects, features, and advantages of the inventive concept will become apparent from the following description of embodiments given in conjunction with the accompanying drawings. However, the inventive concept is not limited to the embodiments disclosed herein and may be implemented in various different forms. Herein, the embodiments are provided to provide complete disclosure of the inventive concept and to provide thorough understanding of the inventive concept to those skilled in the art to which the inventive concept pertains, and the scope of the inventive concept should be limited only by the accompanying claims and equivalents thereof.

Unless otherwise defined, all terms (including technical and scientific terms) used herein have the same meaning as commonly understood by those skilled in the art to which the inventive concept pertains. It will be further understood that terms, such as those defined in commonly used dictionaries, should not be interpreted in an idealized or overly formal sense unless expressly so defined herein.

Spatially relative terms, such as "beneath", "below", "lower", "above", "upper" and the like, may be used herein for ease of description to describe one component or feature's relationship to another component(s) or feature(s) as illustrated in the figures. It will be understood that the spatially relative terms are intended to encompass different orientations of the device in use or operation in addition to the orientation depicted in the figures. For example, if the device in the figures is turned over, components described as "below" or "beneath" other components or features would then be oriented "above" the other components or features. Thus, the exemplary term "below" can encompass both an orientation of above and below. The device may be otherwise oriented (rotated 90 degrees or at other orientations) and the spatially relative descriptors used herein interpreted accordingly.

Hereinafter, embodiments of the inventive concept will be described in detail with reference to the accompanying drawings.

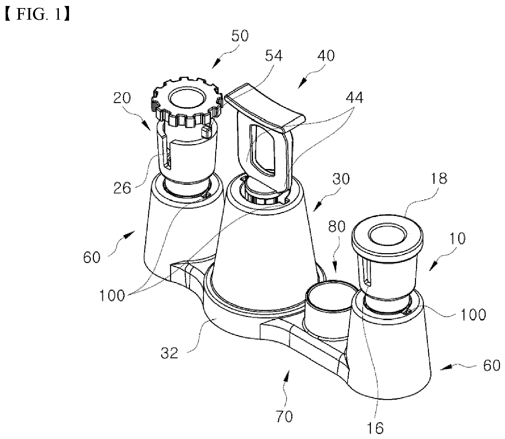

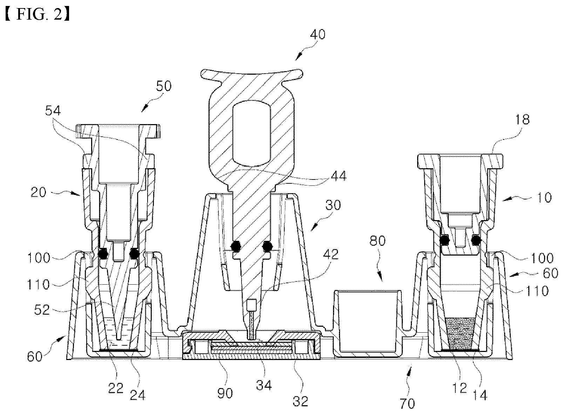

FIG. 1 is a perspective view illustrating a glycated hemoglobin measurement kit according to an embodiment of the inventive concept, and FIG. 2 is a sectional view illustrating the glycated hemoglobin measurement kit according to an embodiment of the inventive concept.

As illustrated in FIGS. 1 and 2, the glycated hemoglobin measurement kit according to an embodiment of the inventive concept may include a reagent container 10, a cleaning solution container 20, a container holder 30, a capillary 40, a breaking member 50, a pair of housings 60, and a base 70.

The reagent container 10 serves to accommodate a reagent for measurement of glycated hemoglobin in blood. Specifically, the reagent container 10 has a cylindrical shape that is open at opposite sides. The reagent container 10 includes a barrier membrane 14 that blocks an opening at one side of the reagent container 10 and forms a reagent storage part 12 in which the reagent for measurement of glycated hemoglobin in blood is accommodated. For example, the reagent container 10 may be open at the lower and upper sides, and the lower opening of the reagent container 10 may be blocked by the barrier membrane 14. Furthermore, the upper opening of the reagent container 10 may be covered by a reagent cover 18. In an embodiment, the upper opening of the reagent container 10 may have a stepped shape, and the reagent cover 18 may have a stepped shape corresponding to the upper opening of the reagent container 10.

In an embodiment, the barrier membrane 14 may be formed of a flexible material. The flexible material may be rubber.

In an embodiment, the reagent storage part 12 may be formed in a lower portion of the reagent container 10, and the reagent may be accommodated in the reagent storage part 12.

In an embodiment, the reagent, which is used to measure glycated hemoglobin in blood, serves to disintegrate red blood cells in the blood to induce a hemolytic phenomenon such that hemoglobin in the blood is eluted out of the blood cells. The reagent may include other effects in addition to the induction of the hemolytic phenomenon. For example, a liquid reagent may be used as the reagent. However, the inventive concept is not particularly limited thereto, and a solid reagent may be used as the reagent.

In an embodiment, the reagent storage part 12 may have a shape with a decreasing diameter toward the lower side. The reagent storage part 12 may be easily inserted into the pair of housings 60 or the container holder 30 to be described below.

The cleaning solution container 20 serves to accommodate a cleaning solution that cleans a reaction solution in which blood and the reagent are mixed and react with each other. Specifically, the cleaning solution container 20 has a cylindrical shape that is open at opposite sides. The cleaning solution container 20 includes an auxiliary barrier membrane 24 that blocks an opening at one side of the cleaning solution container 20 and forms a cleaning solution storage part 22 that accommodates the cleaning solution that cleans the reaction solution in which the blood and the reagent are mixed and react with each other. For example, the cleaning solution container 20 may be open at the lower and upper sides, and the lower opening of the cleaning solution container 20 may be blocked by the auxiliary barrier membrane 24. Furthermore, the upper opening of the cleaning solution container 20 may be covered by the breaking member 50 to be described below.

In an embodiment, the auxiliary barrier membrane 24 may be formed of a flexible material. The flexible material may be rubber.

In an embodiment, the cleaning solution storage part 22 may be formed in a lower portion of the cleaning solution container 20, and the cleaning solution may be accommodated in the cleaning solution storage part 22.

In an embodiment, the cleaning solution serves to clean the reaction solution. When the cleaning solution cleans the reaction solution, this means that the cleaning solution cleans the remaining components other than the hemoglobin contained in the reaction solution.

In an embodiment, the cleaning solution storage part 22 may have a shape with a decreasing diameter toward the lower side. The cleaning solution storage part 22 may be easily inserted into the pair of housings 60 or the container holder 30 to be described below.

The capillary 40 serves to collect blood from a user or break the barrier membrane 14 of the reagent container 10. Specifically, the capillary 40 has a needle 42 that collects blood from the user or breaks the barrier membrane 14 of the reagent container 10. The capillary 40 may be attached to or detached from the reagent container 10, or may be selectively attached to or detached from the container holder 30.

For example, when the capillary 40 is mounted in the reagent container 10, the reaction solution in which the blood and the reagent react with each other may be generated in the reagent storage part 12 as the blood collected in the needle 42 of the capillary 40 is injected into the reagent accommodated in the reagent storage part 12 of the reagent container 10. At this time, the reaction rate of the blood and the reagent may be improved by shaking, by the user, the reagent container 10 having the capillary 40 mounted therein.

Thereafter, when the reagent container 10 having the capillary 40 mounted therein is mounted in the container holder 30 to be described below and the needle 42 of the capillary 40 moves toward the barrier membrane 14 of the reagent container 10 and breaks the barrier membrane 14 of the reagent container 10, the reaction solution may be provided to a measurement strip 90 through a measurement hole 34 of the container holder 30 to be described below.

In an embodiment, to prevent the needle 42 of the capillary 40 from moving toward the barrier membrane 14 of the reagent container 10 and breaking the barrier membrane 14 of the reagent container 10 at a time point not desired by the user, the glycated hemoglobin measurement kit according to an embodiment of the inventive concept may further include a stopper 44 and a guide groove 16.

The stopper 44 may be provided on the capillary 40 and may be supported on the periphery of an opening at an opposite side of the reagent container 10 to limit a movement of the capillary 40. For example, the stopper 44 may protrude from the periphery of the capillary 40 in a radial direction. Furthermore, although not particularly limited, the stopper 44 may include a pair of stoppers.

The guide groove 16 may be formed in the reagent container 10 through cutting and may guide a movement of the stopper 44 such that the capillary 40 moves toward the barrier membrane 14 and the needle 42 of the capillary 40 breaks the barrier membrane 14. In an embodiment, although not particularly limited, the guide groove 16 may include a pair of guide grooves.

In an embodiment, when the stopper 44 and the guide groove 16 are staggered with respect to each other, the stopper 44 may be supported on the periphery of the opening at the opposite side of the reagent container 10 to limit a movement of the capillary 40.

Furthermore, when the stopper 44 and the guide groove 16 are aligned with each other, the stopper 44 moves along the guide groove 16, and as the stopper 44 moves along the guide groove 16, the needle 42 of the capillary 40 moves toward the barrier membrane 14 and breaks the barrier membrane 14.

The breaking member 50 serves to break the auxiliary barrier membrane 24. The breaking member 50 may have an auxiliary needle 52 that breaks the auxiliary barrier membrane 24 and may be attached to or detached from the cleaning solution container 20. In an embodiment, the upper opening of the cleaning solution container 20 may have a stepped shape, and the breaking member 50 may have a stepped shape corresponding to the upper opening of the cleaning solution container 20.

In an embodiment, to prevent the auxiliary needle 52 of the breaking member 50 from moving toward the auxiliary barrier membrane 24 of the cleaning solution container 20 and breaking the auxiliary barrier membrane 24 of the cleaning solution container 20 at a time point not desired by the user, the glycated hemoglobin measurement kit according to an embodiment of the inventive concept may further include an auxiliary stopper 54 and an auxiliary guide groove 26.

The auxiliary stopper 54 may be provided on the breaking member 50 and may be supported on the periphery of an opening at an opposite side of the cleaning solution container 20 to limit a movement of the breaking member 50. For example, the auxiliary stopper 54 may protrude from the periphery of the breaking member 50 in a radial direction. Furthermore, although not particularly limited, the auxiliary stopper 54 may include a pair of auxiliary stoppers.

The auxiliary guide groove 26 may be formed in the cleaning solution container 20 through cutting and may guide a movement of the auxiliary stopper 54 such that the breaking member 50 moves toward the auxiliary barrier membrane 24 and breaks the auxiliary barrier membrane 24. In an embodiment, although not particularly limited, the auxiliary guide groove 26 may include a pair of auxiliary guide grooves.

In an embodiment, when the auxiliary stopper 54 and the auxiliary guide groove 26 are staggered with respect to each other, the auxiliary stopper 54 may be supported on the periphery of the opening at the opposite side of the cleaning solution container 20 to limit a movement of the breaking member 50.

Furthermore, when the auxiliary stopper 54 and the auxiliary guide groove 26 are aligned with each other, the auxiliary stopper 54 may move along the auxiliary guide groove 26. At this time, the breaking member 50 may move toward the auxiliary barrier membrane 24, and the auxiliary needle 52 of the breaking member 50 may break the auxiliary barrier membrane 24.

The reagent container 10, the cleaning solution container 20, and the capillary 40 may be selectively attached to or detached from the container holder 30. The container holder 30 may have a shape protruding from the base 70 to be described below. For example, the container holder 30 may have a shape with a decreasing diameter in the direction in which the container holder 30 protrudes from the base 70 to be described below.

The container holder 30 may be sequentially provided with the reaction solution in which the blood and the reagent are mixed and react with each other and the cleaning solution. Detailed description thereabout will be given below.

The container holder 30 may include an attachment/detachment part 32 from which the measurement strip 90 for measuring glycated hemoglobin is detachable and the measurement hole 34 through which one area of the measurement strip 90 attached to the attachment/detachment part 32 is exposed, and the reagent container 10 or the cleaning solution container 20 may be selectively attached to or detached from the container holder 30 such that the reaction solution or the cleaning solution is selectively provided toward the measurement hole 34.

For example, after the reagent container 10 having the capillary 40 mounted therein and having the reaction solution accommodated in the reagent storage part 12 is mounted in the container holder 30, the reaction solution may be provided to the measurement hole 34 when the needle 42 of the capillary 40 moves toward the barrier membrane 14 of the reagent container 10 and breaks the barrier membrane 14 of the reagent container 10. The reaction solution provided in this way may be stored in the measurement strip 90.

After the reagent container 10 mounted in the container holder 30 is removed and the cleaning solution container 20 is mounted in the container holder 30, the cleaning solution may be provided to the measurement hole 34 when the breaking member 50 breaks the auxiliary barrier membrane 24 of the cleaning solution container 20. The cleaning solution provided in this way may be stored in the measurement strip 90 and may clean the reaction solution.

A membrane in which the mixture of the reaction solution and the cleaning solution supplied from the measurement hole 34 is stored may be accommodated in the measurement strip 90. The measurement strip 90 may be removed from the attachment/detachment part 32 and may be inserted into a glycated hemoglobin measurement device 120, and the glycated hemoglobin of the mixture stored in the measurement strip 90 may be measured by the glycated hemoglobin measurement device 120.

The reagent container 10 or the cleaning solution container 20 may be accommodated in the pair of housings 60 so as to be selectively detachable. The pair of housings 60 may have a shape protruding from the base 70 to be described below. For example, the pair of housings 60 may have a shape with a decreasing diameter in the direction in which the pair of housings 60 protrude from the base 70 to be described below.

The base 70 may support the pair of housings 60 and the container holder 30.

In an embodiment, the container holder 30, the pair of housings 60, and the base 70 may be integrally combined to form a cartridge.

A cover storage part 80 for storing the reagent cover 18 may be provided on the cartridge. Accordingly, the user may store the reagent cover 18 in the cover storage part 80 as needed.

Meanwhile, the glycated hemoglobin measurement kit according to an embodiment of the inventive concept may further include first alignment parts 100 and second alignment parts 110. When the reagent container 10 or the cleaning solution container 20 is selectively mounted in a selected one of the housings 60 and the container holder 30, the first alignment part 100 and the second alignment part 110 may serve to align the reagent container 10 or the cleaning solution container 20 with the selected one of the housings 60 and the container holder 30.

The first alignment parts 100 may be concavely formed on the inner circumferential surfaces of the housings 60 and the container holder 30 along the central axis directions of the housings 60 and the container holder 30. For example, the housings 60 and the container holder 30 may each include a plurality of first alignment parts 100. The plurality of first alignment parts 100 may be radially formed on the inner circumferential surfaces of the housings 60 and the container holder 30.

The second alignment parts 110 may protrude from the outer circumferential surfaces of the reagent container 10 and the cleaning solution container 20 and may be engaged with the first alignment parts 100. For example, the reagent container 10 and the cleaning solution container 20 may each include a plurality of second alignment parts 110. The plurality of second alignment parts 110 may be radially formed on the outer circumferential surfaces of the reagent container 10 and the cleaning solution container 20.

Although FIG. 2 illustrates one example that the first alignment parts 100 are concavely formed on the inner circumferential surfaces of the housings 60 and the inner circumferential surface of the container holder 30 and the second alignment parts 110 protrude from the outer circumferential surfaces of the reagent container 10 and the cleaning solution container 20, the inventive concept is not limited thereto. For example, the first alignment parts 100 may protrude from the inner circumferential surfaces of the housings 60 and the container holder 30, and the second alignment parts 110 may be concavely formed on the outer circumferential surfaces of the reagent container 10 and the cleaning solution container 20.

Hereinafter, a process of measuring glycated hemoglobin using the glycated hemoglobin measurement kit according to an embodiment of the inventive concept will be described. The process may be performed by a controller, such as a microcomputer, or a user.

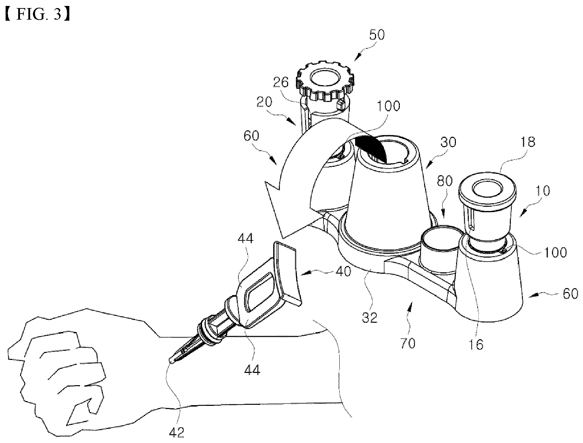

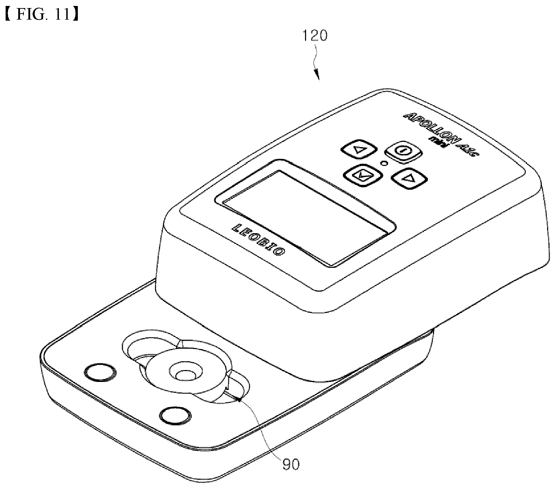

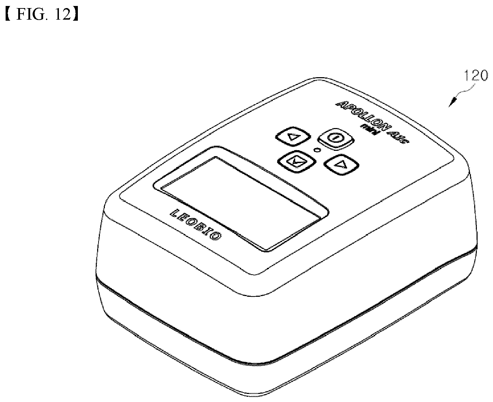

FIGS. 3 to 10 are perspective views illustrating the glycated hemoglobin measurement kit according to an embodiment of the inventive concept, and FIGS. 11 and 12 are perspective views illustrating the glycated hemoglobin measurement device 120 according to an embodiment of the inventive concept.

As illustrated in FIGS. 1 and 2, the reagent container 10 and the cleaning solution container 20 having the breaking member 50 mounted therein are mounted in the pair of housings 60, respectively, and the capillary 40 is mounted in the container holder 30.

As illustrated in FIG. 3, the capillary 40 is removed from the container holder 30, and the user's blood is collected through the needle 42 of the capillary 40. At this time, the blood collected from the user is stored in the needle 42 of the capillary 40.

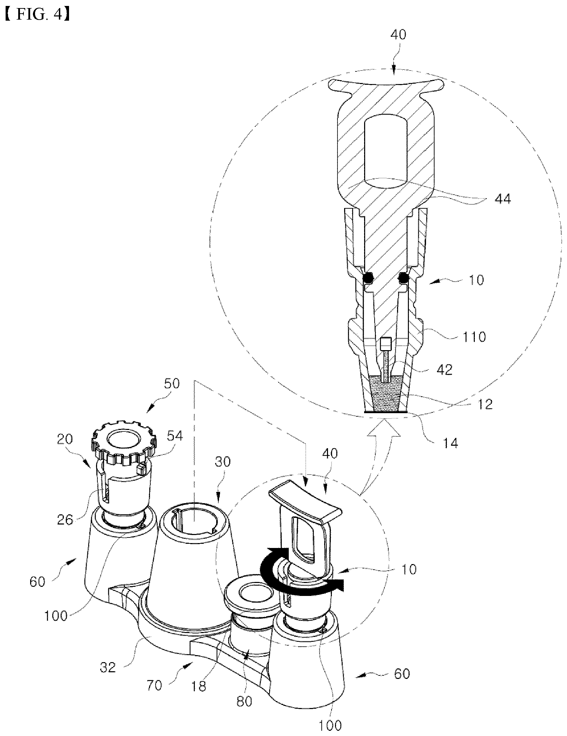

As illustrated in FIG. 4, the reagent cover 18 is stored in the cover storage part 80, and the capillary 40 is mounted in the reagent container 10. As a result, the blood collected in the needle 42 of the capillary 40 is injected into the reagent accommodated in the reagent storage part 12 of the reagent container 10, and a reaction solution in which the blood and the reagent react with each other is generated in the reagent storage part 12. At this time, the reaction rate of the blood and the reagent may be improved by shaking, by the user, the reagent container 10 having the capillary 40 mounted therein.

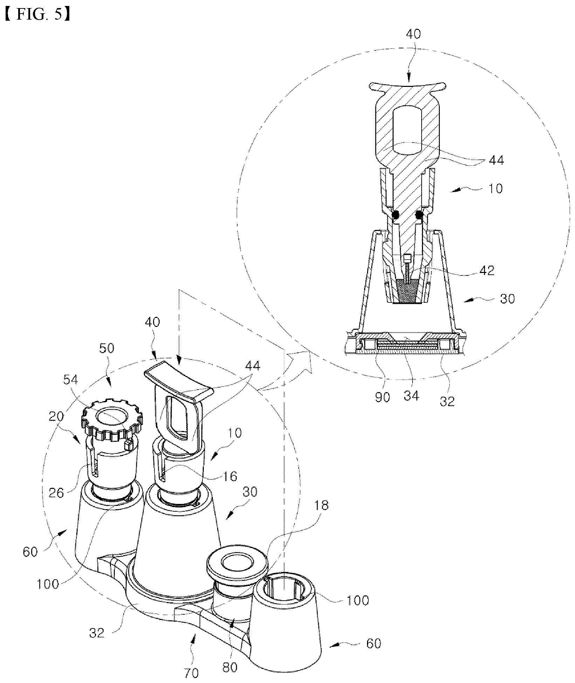

As illustrated in FIG. 5, the reagent container 10 having the capillary 40 mounted therein is mounted in the container holder 30. At this time, the stopper 44 of the capillary 40 and the guide groove 16 of the reagent container 10 are staggered with respect to each other, and thus a movement of the capillary 40 toward the reagent container 10 is limited.

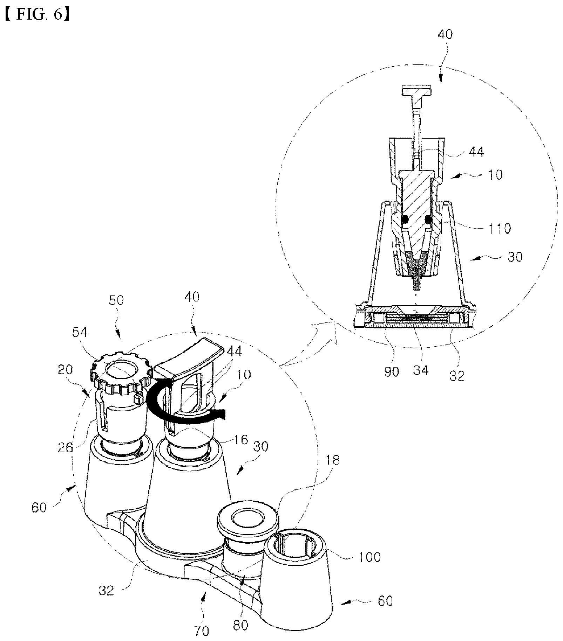

As illustrated in FIG. 6, the stopper 44 of the capillary 40 and the guide groove 16 of the reagent container 10 are aligned with each other by rotation of the capillary 40. Thereafter, the stopper 44 of the capillary 40 moves along the guide groove 16, and the needle 42 of the capillary 40 also moves toward the barrier membrane 14 to penetrate and breaks the barrier membrane 14. As a result, the reaction solution accommodated in the reagent storage part 12 of the reagent container 10 is supplied to the measurement strip 90 through the measurement hole 34 and stored in the measurement strip 90.

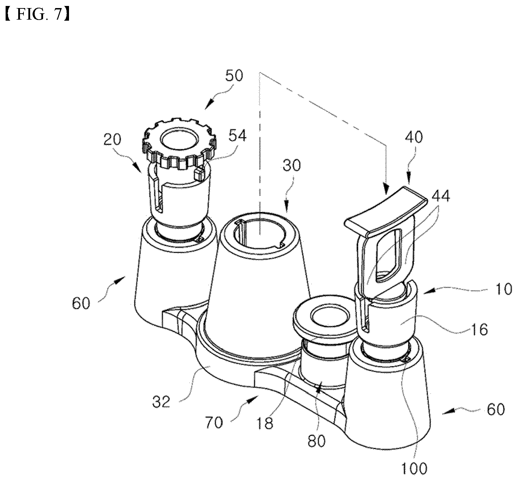

As illustrated in FIG. 7, the reagent container 10 having the capillary 40 mounted therein is mounted in the housing 60.

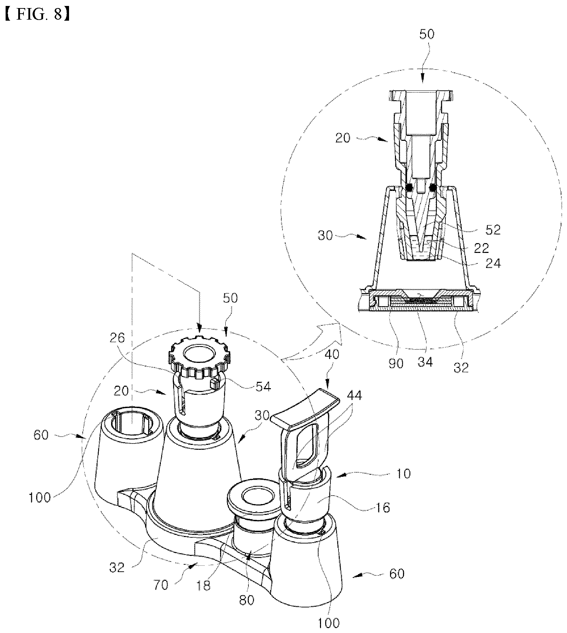

As illustrated in FIG. 8, the cleaning solution container 20 having the breaking member 50 mounted therein is mounted in the container holder 30. At this time, the auxiliary stopper 54 of the breaking member 50 and the auxiliary guide groove 26 of the cleaning solution container 20 are staggered with respect to each other, and thus a movement of the breaking member 50 toward the cleaning solution container 20 is limited.

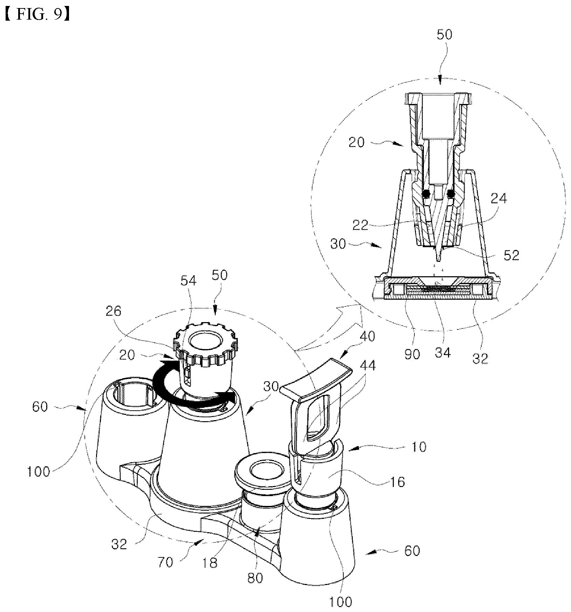

As illustrated in FIG. 9, the auxiliary stopper 54 of the breaking member 50 and the auxiliary guide groove 26 of the cleaning solution container 20 are aligned with each other by rotation of the breaking member 50. Thereafter, the auxiliary stopper 54 of the breaking member 50 moves along the auxiliary guide groove 26 of the cleaning solution container 20, and the auxiliary needle 52 of the breaking member 50 also moves toward the auxiliary barrier membrane 24 to penetrate and breaks the auxiliary barrier membrane 24. As a result, the cleaning solution accommodated in the cleaning solution storage part 22 of the cleaning solution container 20 is supplied to the measurement strip 90 through the measurement hole 34 and stored in the measurement strip 90. At this time, the cleaning solution may clean the remaining components other than the hemoglobin contained in the reaction solution.

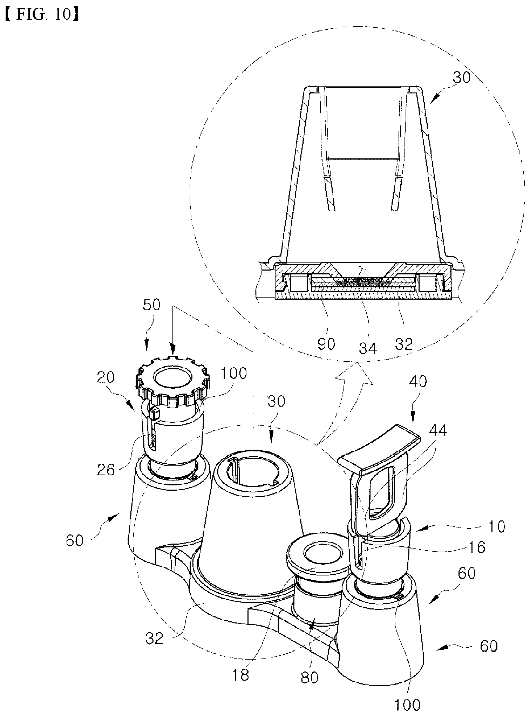

As illustrated in FIG. 10, the cleaning solution container 20 having the breaking member 50 mounted therein is mounted in the housing 60.

As illustrated in FIG. 11, the measurement strip 90 is inserted into the glycated hemoglobin measurement device 120.

As illustrated in FIG. 12, the glycated hemoglobin measurement device 120 measures glycated hemoglobin of the reaction solution stored in the measurement strip 90 and displays the glycated hemoglobin level on a display of the glycated hemoglobin measurement device 120.

The glycated hemoglobin measurement device 120 may be configured such that the upper part and the lower part slide relative to each other.

According to the inventive concept, the glycated hemoglobin measurement kit enables the user to measure the glycated hemoglobin by mixing the blood, the reagent, and the cleaning solution without a visit to a medical institution and is easy to carry.

While the inventive concept has been described with reference to embodiments, it will be apparent to those skilled in the art that various changes and modifications may be made without departing from the spirit and scope of the inventive concept. Therefore, it should be understood that the above embodiments are not limiting, but illustrative.

## Claims

1. A glycated hemoglobin measurement kit comprising:
a reagent container having a cylindrical shape that is open at opposite sides, the reagent container including a barrier membrane configured to block an opening at one side of the reagent container and form a reagent storage part configured to accommodate a reagent for measurement of glycated hemoglobin in blood;
a cleaning solution container having a cylindrical shape that is open at opposite sides, the cleaning solution container including an auxiliary barrier membrane configured to block an opening at one side of the cleaning solution container and form a cleaning solution storage part configured to accommodate a cleaning solution configured to clean a reaction solution in which the blood and the reagent are mixed and react with each other; and
a container holder including an attachment/detachment part from which a measurement strip configured to measure the glycated hemoglobin is detachable and a measurement hole through which one area of the measurement strip attached to the attachment/detachment part is exposed, wherein the reagent container or the cleaning solution container is selectively attached to or detached from the container holder to selectively provide the reaction solution or the cleaning solution toward the measurement hole.

2. The glycated hemoglobin measurement kit of claim 1, further comprising:
a capillary having a needle configured to collect the blood or break the barrier membrane, the capillary being attached to or detached from the reagent container or selectively attached to or detached from the container holder.

3. The glycated hemoglobin measurement kit of claim 1, further comprising:
a breaking member having an auxiliary needle configured to break the auxiliary barrier membrane, the breaking member being attached to or detached from the cleaning solution container.

4. The glycated hemoglobin measurement kit of claim 2, further comprising:
a stopper provided on the capillary and supported on a periphery of an opening at an opposite side of the reagent container to limit a movement of the capillary.

5. The glycated hemoglobin measurement kit of claim 2, further comprising:
a guide groove formed in the reagent container through cutting and configured to guide a movement of the stopper such that the capillary moves toward the barrier membrane and breaks the barrier membrane.

6. The glycated hemoglobin measurement kit of claim 3, further comprising:
an auxiliary stopper provided on the breaking member and supported on a periphery of an opening at an opposite side of the cleaning solution container to limit a movement of the breaking member.

7. The glycated hemoglobin measurement kit of claim 6, further comprising:
an auxiliary guide groove formed in the cleaning solution container through cutting and configured to guide a movement of the auxiliary stopper such that the breaking member moves toward the auxiliary barrier membrane and breaks the auxiliary barrier membrane.

8. The glycated hemoglobin measurement kit of claim 1, further comprising:
a pair of housings in which the reagent container or the cleaning solution container is selectively accommodated so as to be detachable; and
a base configured to support the pair of housings and the container holder.

9. The glycated hemoglobin measurement kit of claim 8, further comprising:
first alignment parts formed to protrude from, or concavely formed on, inner circumferential surfaces of the housings and the container holder along central axis directions of the housings and the container holder; and
second alignment parts formed to protrude from, or concavely formed on, outer circumferential surfaces of the reagent container and the cleaning solution container, the second alignment parts being engaged with the first alignment parts.
